# EUROPEAN PATENT APPLICATION

(11) **EP 1 329 835 A1**
(43) Date of publication of application: **23.07.2003**
(21) Application number: 02001489.0
(22) Date of filing: 22.01.2002
(51) Int. Cl.: G06F 19/00

(54) **Method and system for anonymously administrating medical test results and user-enabled health risk assessment**

(71) Applicant: Genonyme GmbH, 63225 Langen (DE)
(72) Inventor: Bicker, Uwe Prof.Dr.Dr., 64625 Bensheim Auerbach (DE); Pfeiffer, MatthiasDr., 80799 München (DE)
(74) Representative: Harrison, Robert J., Ph. D.

(57) **Abstract**

The application relates to a method and apparatus for anonymously administering at least one test for one or more users. The method comprises a first step of registering (210) the users by recording, in a first repository (90), their identity data. In a next step, a kit is forwarded (230) to each of the users without referencing the identity data. The kit comprises (i) instructions on and (ii) materials for taking a sample for said test. A sample is received (240) from each of the users and a result of said test is obtained on said sample. In a second repository (50) the result for each of said users is recorded (270) under a unique identification code. The first repository (90) and the second repository (50) are insulated from each other.

## Description

### BACKGROUND

This disclosure describes specifically and in general personal information systems, health care systems and systems for the administration of diagnostic and genetic tests. Embodiments disclosed herein relate to user-oriented health management and individualized diagnostics and therapeutics. Other embodiments provide methods and systems for substantially or completely anonymously administering a test - particularly a genetic, diagnostic, or physical test - on one or more users; and methods and systems for enabling a user to make personal health risk assessment in a substantially secured manner.

The completion of the working draft of human genome sequence has marked a significant milestone in man's everlasting endeavor to decode life. It is estimated that the total number of expressed genes or transcription units in a human genome is around 30,000- 40,000 (Venter G. et al., Science 2000, Vol, 291: 1304-1351; The Genome International Sequencing Consortium, Nature 2000, Vol. 409: 860-921). The number of human proteins is expected to significantly exceed this estimate because, in many cases, more than one RNA splice variant is transcribed from a transcription unit or a gene. Estimates vary, but some researchers believe that there exist up to 500,000 human RNA transcripts and that, more than 30% of genes or transcription units in the human genome produce several RNA splice variants. (Mironov et al. 1999, Genome Research 9:1288-1293). Considering further the individual genomic diversities in the human species, evidenced, for example, by an increasing number of single nucleotide polymorphisms ("SNPs") identified, this immense genomic data pool lays the foundation for a better understanding of human physiology and brings about significant therapeutic and diagnostic promises.

Of particular relevance is individual genetic predisposition, a challenging area to biomedical researchers, genetic counselors, as well as clinicians. It is estimated that the DNA sequence of any two given human beings differ by approximately 0.1%. These differences in DNA base composition can result in different protein functions, and thus become consequential with respect to the individual's physical well-being in some situations.

Recent years have seen dramatic growth in the information of genetic predisposition, the responsible genes, and their specific mutations. More mutations are being detected which are connected to higher risks of developing certain diseases, often with an earlier onset. For example, there is an increased total number of entries in the Mendelian Inheritance in Man (which has more than 10,000 entries as to date). See also, the Online Mendelian Inheritance in Man database (OMIM) at http://www3.ncbi.nlm.nih.gov/Omim/searchomim.html. As consequences of genetic predispositions are better appreciated today, techniques for genetic diagnosis are made available in clinical settings. An increasing number of genetic tests are offered to measure the potential mutations. As of 1999, 667 different genetic tests (excluding DNA-analysis tests for infectious diseases) were offered, up from 111 in 1993 (GeneTests.org, Washington G2-Reports: Lab Industry Outlook 2000 as cited in Merril Lynch, In Vitro Diagnostics, 14 November 2000).

Whereas advances are being made to find cures for some genetic diseases, many of them do not yet have effective therapies. However, lifestyle and dietary avoidance strategies are becoming feasible for a significant number of genetic diseases, which makes it extremely critical and beneficial to detect genetic predispositions early in time. That is, if an individual knows that a disease-responsible gene - a gene that is involved in the development, onset, or progression of a disease - carry mutations in his or her genome, he or she may institute recommended changes in lifestyle and diet to postpone or avoid the outbreak of the disease. Therefore, the diagnostic and therapeutic promises of the human genome data may be realized as the data is transformed into personalized knowledge on individual genes and their impact on the onset and severity of a disease.

Predisposition to adverse drug response is similarly of importance. Adverse drug reaction is one of the leading causes of death in hospitalized patients worldwide. It is recognized as the fourth to fifth leading cause of death in hospitalized patients in the United States. Of more than two million Americans hospitalized each year for adverse drug reactions, over 100,000 people die (JAMA 79, 1200 -1205 (1998)). Genetic predisposition testing is rather promising in potentially eliminating these deaths because heritability (or the genetic component) plays a large part in adverse drug response. Prediction of drug response therefore would have profound impact on the delivery of personalized therapeutics, a goal sets out by the new evolving discipline, pharmacogenomics.

In sum, there is a huge and increasing demand for genetic tests - or other diagnostic and physical tests, for that matter - propelled by the new genomic knowledge and technology capacities, as well as individuals' desire to be better self-informed of health risks and their desire to engage in proactive personal health management. A recent poll in the U.S. and Europe indicate that approximately 2/3 of all people in western societies would like to know their genes and their genetic predisposition.

However, to meet such demand and to empower individuals to engage in proactive personal health management, certain issues must be addressed, including especially the issue of privacy. People generally fear that genetic information may be made available to third parties - such as employers and insurers - and used to their detriment. In order to address this concern, an appropriate or reasonable level of privacy must be ensured, advantageously including high-standard security and anonymity.

### BRIEF DESCRIPTION OF DRAWINGS

Fig. 1 depicts a computer system for anonymously administering one or more tests and for enabling personalized health risk assessment according to one embodiment of disclosure.
Fig. 2 depicts a flow diagram illustrating the method for anonymously administering one or more tests.
Fig.3 depicts an encrypted e-mail as sent over the network with the details of the order.

### DETAILED DESCRIPTION OF DISCLOSED EMBODIMENTS

### Privacy, Security And Anonymity

A feature of certain embodiments of the methods and systems of the present disclosure is a reasonable level of privacy. Such a level of privacy is achieved by instituting predetermined levels of security and anonymity in the involved structural components and dynamic processes, thereby enabling users to perform one or more tests of interest, establish and manage personal health profiles, make health risk assessments, and seek further medical counseling as needed.

The test results are stored into a repository, also referred to as a databank; it is preferably an electronic repository such as a database. Analogized to a Swiss bank, the databank is highly secured, having completely anonymous electronic accounts for the user. A user defines or is assigned his own unique password which is known only to the user and not accessible to any third party, including the data bank operator. While a password generally will be the easiest method for a user to identify himself or herself, it is expressly understood and contemplated that the skilled artisan could substitute for a password other possible user recognition hardware, software, methods and/or systems, including for example, userIDs, biometric and behavioral recognition systems such as fingerprint, voice, face, retina and signature recognition systems, smartcards, badges, and other electronic and digital signature systems, to name only a few. Likewise, combinations of the foregoing could be used. Hence, unless indicated to the contrary, a reference to the use of a password in this disclosure is understood to include by reference the interchangeable use of one or more of any such means of recognition.

In the embodiments disclosed herein, encryption can be implemented for data transfer into and out of the databank. The databank of such embodiments thus can be reasonably highly safeguarded against user-identity hacking. In extremely unlikely cases where hackers break into the databank, the identities of the users will still remain inaccessible if there is substantial or complete anonymity of the databank. Thus, embodiments disclosed herein can provide users with a reasonable level of privacy especially if a high level of security is coupled with a high level of anonymity.

In a variant of this embodiment, the databank can store genetic or other data for a user, which data he or she has obtained from other sources. For example, the user may have some test results from earlier tests performed in other places or contexts, and these results can be transferred by the user to the databank and stored within his or her record under the unique identification code.

Additional repositories can be employed in other embodiments to store and manage other pertinent information, including, inter alia, users' personal information, their family history and medical history, general medical background information on specific disorders or abnormal conditions, and information on the related diagnostics and therapies. These repositories may be implemented with lower or higher privacy level (typically lower) as desired. For example, in a particular embodiment, a first repository is used which has stored therein the user identity information. In this embodiment, the aforementioned databank is then referred to as the second repository. In contrast to the second repository, the first repository does not require anonymity or strict security measures. Rather, its main objective is to keep track of the users, and permit users to as they register with the system and order the necessary sampling kit for one or more genetic or diagnostic tests.

In another embodiment, and as a further example, a third repository can be employed which is a medical knowledge base. This third repository can include, for example, general biomedical background information on human physiology, diseases, diagnosis and therapies, information on users' medical history, information on medical practitioners and specialists, among other things. The third repository, as all the other repositories employed according to this disclosure, is preferably a database and linked to an interface through which the user may input instructions such as search queries and view the output results. In one embodiment, on his or her own command, a user may choose to transfer his or her test results, which is stored at the first instance in the aforementioned databank in an absolute anonymous and secured fashion, to the third repository, thereby allowing more extensive analysis and/or counseling on his or her health conditions. Such further analysis and/or counseling may be sought, for example, from a medical practitioner having certain special expertise of interest, identified by the user from the information presented by the third repository. The counseling may include advice on new medication, avoidance of certain drugs, specific diets and life style recommendations, for example. Alternatively, such further analysis and counseling may be obtained as the user queries the third repository through the interface and reviews pertinent information retrieved therefrom. Depending upon its purpose and function, the third repository can operate with different levels of privacy or even no privacy, and may not require the same level of security and/or anonymity as the second repository, i.e., the databank, which normally has high security and/or anonymity.

Yet other embodiments employ a fourth repository which has stored therein the test samples from the users, or any biological molecules such as DNAs, RNAs, and proteins extracted therefrom. In one embodiment, these samples may be sent to the fourth repository only upon the users' express instruction. Without such instruction, the test samples will be destroyed immediately after the ordered tests are performed. If a user does request his or her sample to be kept and deposited to the fourth repository, he or she can take one or more additional steps with the sample, for example, order an additional test or rerun the ordered test as desired. Similar to the records in the second repository, or the databank, deposits in the fourth repository can be anonymous, for example, identifiable only by the unique password of the submitting user. A predetermined level of security (typically high) also can be implemented for the fourth repository. Therefore, a user's privacy could be safeguarded by a predetermined level of security if he or she elects to keep their test samples for future use or reference.

Therefore, different levels of security and privacy are provided for various information and sample repositories according to embodiments disclosed herein to serve different functions and purposes. Moreover, as discussed below, the level of privacy the users enjoy with respect to their test results and test samples not only relies on the levels of anonymity and/or security of the repositories, but also can turn on one or more other characteristics of the systems and methods disclosed herein. That is, certain repositories can be insulated from other repositories, as desired. For example, in the embodiment above, the first and the second repositories can be substantially insulated from each other and, similarly, the first and the fourth repository can be substantially insulated from each other. Thus, repositories where higher levels of privacy and/or security are desired can be insulated from those that may operate with less stringent requirements of privacy and security.

The term "insulation" or "insulate" as used herein, refers to the substantially separate and independent existence and operation of the selected repositories, whether physically, electronically, or network-wise. Advantageously, there is complete detachment, whether physically, electronically, or network-wise. For example, a user may have entries in all the repositories, but he or she is only identified in the second and fourth repositories - the repositories endowed with higher levels of privacy - by a unique password created (or assigned) and controlled by him or herself. The unique password works with a unique identification code which may, for example, either be created by the user and communicated to the operator of the system or created by the operator of the system and communicated to the user anonymously. As such, the true identity of the user can be concealed from the operator of the system and yet the system is able to safely deliver the test result to the databank under the unique identification code which the user is able to track down and match with his or her unique password to thereby retrieve the results. Such retrieval can only be done by the user or a designee thereof who possesses both the unique identification code and the unique password.

### Computer System for the Implementation of the Method

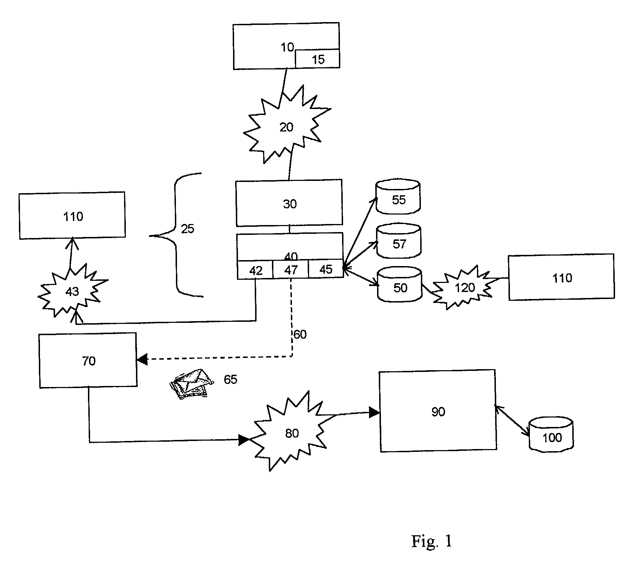

An example of an embodiment for the administration of the test is shown in Fig. 1. The computer system comprises a personal computer 10 at which the user of the test can order the test and through which the user of the test can retrieve the results of the test. The personal computer 10 includes a display device and runs a browser 15, such as Netscape Navigator or Microsoft Internet Explorer, which accesses a remote computer 25 through a network 20. The network 20 can be a local area network or a virtual private network (VPN), but is more preferably the internet.

The remote computer 25 is running a network interface program 30 such as the Microsoft Internet Interface Server or an Apache Server. The interface program 30 accepts instructions from the network 20 and passes them through to a test administration program 40 which is running on the remote computer 25.

In one embodiment of the invetion, the interface program 30 uses the HTTPS protocol according to SSL level 2 or 3 to transfer the instructions from the personal computer 10 to the remote computer 25. Alternatively, VPN-on-demand technology can be used to set up an encrypted channel over the network 20 between the personal computer 10 and the remote computer 25.

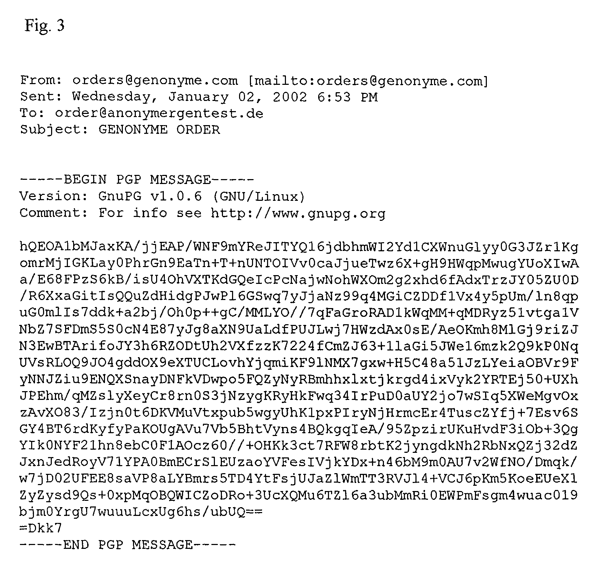

The test administration program 40 includes a database server 45 connected to a databank 50, which holds the results of the tests, a product database 52, which holds details of the tests that may be ordered, and an e-mail administration module 47 which is adapated to send e-mails over a further network 60. The database server 45 can be a database program such as MySQL, Microsoft Access, the ORACLE database system obtainable from Oracle, the Objectivity/DB system obtainable from Objectivity, Inc., or IBM DB2. The database server 45 is connected to a databank 50. The databank 50 can be stored in dynamic or static memory and, for faster access, may include caching systems as is known to the skilled person. The test administration program 40 also includes an encryption program which is used to encrypt any data sent from the personal computer 10 to the remote computer 25 and for encrypting e-mails in the e-mail administration module 47. An example of an encrypted e-mail is shown in Fig. 3.

The database server 45 may also be connected to an annotation storage 57 which function will be explained later. The remote computer 25 may further include a knowledge base 55 or be linked through the network 20 to a knowledge base 55. The function of the knowledge base 55 will be explained later.

The remote computer 25 further includes a payment administration module 42 which is connected to a financial institution, such as the EasyCash company in Germany. The payment administration module 42 takes details of the method of payment desired by the user of the test and passes it through a network 43 to the financial institution 44 for debiting from the user's bank or credit card account.

As will be explained in more detail later, the e-mail administration module 47 sends any data related to a user's order through the further network 60 to an intermediate storage area 70. In one embodiment of the invention, three intermediate storage areas 70 are used to ensure that no data is lost in the event of a breakdown of one of the intermediate storage areas.

A local server 90 is provided in a secure facility on which the details of the user's order are stored in a local storage 100. The local storage 100 only includes names and addresses of the users and will not hold test results. The local server 90 is connected to the intermediate storage area 70 through a network 80. The network 80 can be the internet. In the preferred embodiment of the invention, the local server 90 is not permanently connected to the intermediate storage 70 but is only connected on-demand for short periods of time to download the data on the intermediate storage 70. After downloading of the data from the intermediate storage 70, the local server 90 is disconnected from the network 80 and the data deleted from the intermediate storage 70. The local server 90 can run any suitable operating system, e.g. Microsoft Windows or Linux, and network connection program. The local server 90 is furthermore provided with an e-mail module and encryption software to enable it to read the e-mails downloaded from the intermediate storage 70. Decryption of the e-mails is only carried out when the local server 90 is off-line in order to ensure that access to the decrypted information is restricted. The local server 90 is furthermore provided with the necessary software to ensure that the user's order is processed.

A testing center 110, comprising a laboratory and a computer system, is shown in the figures. The testing center 110 performs the tests in an anonymous manner and returns the results of the tests over a network 120 to the databank 50.

In addition to the Internet access through the network 20, the system also offers telephone access to the system. Registration may also be done by sending a postcard or letter to the administer/operator of the system. Pre-made registration postcards may be distributed by the operator/administer of the system to facilitate user registration, which include check boxes for various tests offered, the methods of payment, and for requesting additional information on certain tests and services provided. The credit card information may also be transferred over telephone. Upon registration, a telephone number is provided to the user for future access of his or her test results, using, like the Internet access, the unique identification code.

### Ordering & Administation of the Test

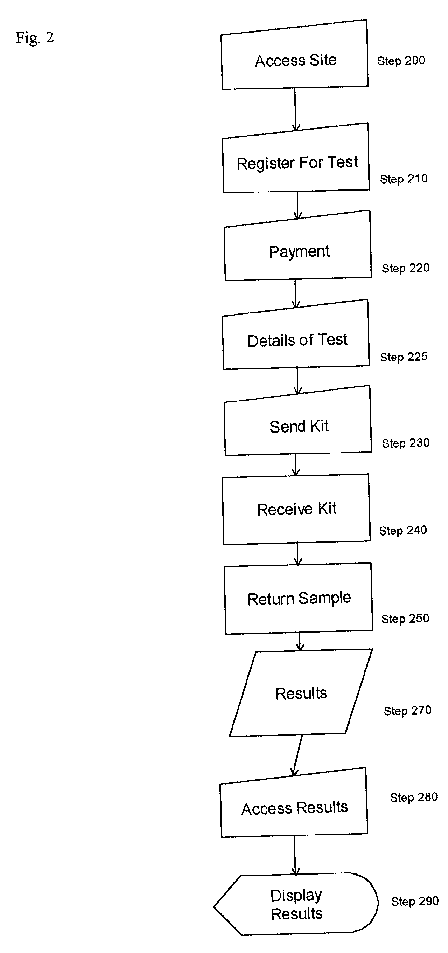

The privacy, anonymity, and security properties of some of the embodiments discussed herein be further appreciated, for example, by examining the depiction of Fig. 2. Fig. 2 provides an overview of certain embodiments for ordering and anonymously administering one or more tests and for enabling personalized health risk assessment. Specifically, the following steps or processes may be performed.

In the first step 200, one or more users access a portal on the remote computer 25 at which information about the test can be viewed and the test can be ordered. Access to the portal is obtained by entering an IP address or a domain name in the browser 15 on the personal computer 10. The browser 15 accesses through the network 20 the remote computer 25 on which the test administration program 40 is running. The user at the personal computer 10 can access details of the tests which can be ordered through database server 45 which requests details of the tests stored in the product database 52 and returns these details in a displayable form to the browser 15.

In step 210, the user registers for the test. The registration is carried out by the user filling out a form displayed on his browser 15. This form includes the name and address of the user and the method of payment which the user chooses. The form may further include an acknowledgement of the general terms and conditions of the provider of the test and a liability exclusion.These details, together with the order number or name of the test, are encrypted using the encryption technology provided in the browser 15 and on the remote computer 30 and transferred through the network 20 to the remote computer 30.

At the remote computer 30, payment for the test is initiated in step 200. Processing of the payment is carried out in the payment administration module 42 as explained above. In step 225, details of the order are forwarded to the test provider. This is done by the test administration software 40 which co-operates with the E-mail administration module 47 to generate an encrypted e-mail 65 (such as shown in Fig. 3) which is passed through the network 60 to the intermediate storage 70. Details of the user and the test which has been ordered are not permanently maintained on the remote computer 30. Instead of an encrypted e-mail, encrypted data sets may be transferred.

In step 230, the test kit is sent to the user. The local computer 90 downloads at periodic intervals from the intermediate storage 70 the encrypted e-mails 65 and disencrypts them. The local computer 90 then generates a delivery note for the test kit dispatchers. The local computer 90 also generates a unique identity number for each each user. The unique identity number is a ten digit number and is sent with the test kit to the user.

The test kit dispatcher forwards to the user a kit or package which contains materials for taking a sample for the ordered test and instructions on how to collect the sample. The materials may include, among other things, sample tubes, antiseptic agents, and syringes. In one of the alternative embodiments, the kit further contains a unique identification code which has been created by the system, i.e., the operator or administer of the system, randomly and anonymously. Along with the kit, the unique identification code is sent to the user anonymously, that is, without any reference to the user's identity. In other alternative embodiments, the unique identification code may be created directly by the user and therefore remain known only to the user.

In step 240, the user receives the kit together with his or her unique identitification code. The user takes a sample and returns the sample in step 250 to the testing center 110 who then analyses the sample and in step 260 provides the results of the test together through the network 120 with the unique identitification code to the databank 50 on the remote computer 25.

Samples may be any sample containing genetic material, including for example, blood or blood components, palatal swaps (i.e., epithelial cells extracted from the mouth cavity), urine, and other human non-blood genetic material, among other things. In the alternative embodiment where the unique identification code is created by the user, this code is transferred to the system or operator/administer of the system along with the sample, such that the test results may be recorded later under the corresponding code. It is to be noted that the test samples and test results are only identified with the unique identification code known to the user; the system or operator/administer of the system has no access to the code and no way of linking the test results to the true identity of a registered user. Such anonymity and privacy guard is enabled by the insulation between the databank 50 and the first repository on the local computer 90 as discussed supra.

The test results may be accessed and reviewed only by the user, through a secured interface linked to the second repository.The user can access the results of the test in step 270 by means of the browser 15 and the database server 45 on the remote computer. The user enters his or her unique identification number in the browser 15 which acts as a validator by encrypting the number and passing it to the test administration program 40. The test administration program decodes the unique identification number and passes it to the database server 45 which accesses the test results in the result memory 55 and returns it to the test administration program 40. The test administration program 40 then passes the results to the browser 15 for display to the user in step 280.

The forgoing steps 210 to 290 allow substantially anonymous administration of one or more tests ordered by a user. To enable a user to make health risk assessment based on these test results, certain additional steps can be performed as discussed below.

### User-Enabled Health Risk Assessment

In other embodiments, one or more systems and methods for enabling a user to assess heath risks are added to the above-described systems and methods. Skilled artisans will recognize many possible systems and methods for enabling a user to assess health risks on the basis of the test results obtained. A few examples are described below, but many others are readily possible.

One embodiment employs a classification schema based on the tests ordered by the user, which comprises a plurality of risk classes ranked from the lowest risk to the highest risk, relating to the one or more disorders of interest. The user may make personal health risk assessment with respect to one or more disorders by determining a risk class based on his or her test results.

For example, for a test that examines the sequences of one or more genes contributing to a disorder, a classification schema may comprise the following risk classes:
(i) risk class I, no increased genetic risk of developing the disorder. This class represents the homozygous wild type group; both alleles of the gene are normal. Usually this is the best class to be in.
(ii) risk class II, usually moderately increased relative genetic risk of developing the disorder. This class represents the heterozygous wild type - mutant group; one allele of the gene is mutated.
(iii) risk class III, usually moderately to severely increased relative genetic risk of developing the disorder. This class represents the homozygous mutant group; both alleles of the gene is mutated.
(iv) risk class IV, usually highly increased relative genetic risk of developing the disorder. The class represents the combination of several mutations in different genes contributing to the disorder; different alleles of more than one gene are mutated. This typically is the worst class to be in.

A risk class can be further divided into subclasses to take into account, for example, the environmental or behavior factors, such as smoking, anti-contraceptives, overweight, and immobilization. Skilled artisans readily will recognize various other possible classification schemes that are possible for use in such embodiments.

Consistent with the classification schema, the test results may be annotated by the system and stored in the annotation storage 57 to assist in the user's understanding and interpretation of the results. Based on these results and annotation - if any - presented by the system, through the interface linked to the databank 50, the user will be able to determine a risk class for him or herself with respect to the disorder of interest. As such, a lay person with no special medical knowledge, according to this disclosure, will be able to self-classify his or her relative health risks and to follow suggested avoidance strategies without consulting a medical professional. Further professional consultation may be sought, however, as discussed above, if the user so desires. Typical situations for pursuance of further diagnostic or therapeutic assistance are those where a high risk class has been identified. Therefore, by enabling self-assessment of health risks, the present disclosure empowers a user or a patient to act proactively in monitoring his or her health conditions and take preventative measures in managing potential health problems.

### Anonymous and Secured Repository For Genetic Samples

In other embodiments, a fourth repository is employed in the methods and systems discussed above. This repository may be considered as a secured sample repository where samples such as genetic materials of a user are kept substantially or completely anonymously, e.g., marked with a unique identification code or a unique barcode known only to the user.

The deposition of sample materials in the fourth repository may only be authorized and effectuated by the user. For example, absent the user's express request, which may be transferred to the system through an interface such as the interface of the second repository (i.e., the databank 55), the test samples can be destroyed after the ordered tests are performed. If a user elects to keep the test samples (or the biological molecules such as DNAs, RNAs, and proteins extracted therefrom), the samples (or the extracted DNAs, RNAs, and proteins) can be forwarded, following the completion of the ordered tests, to the fourth depository and stored, advantageously in a highly secured manner, under one or more identification codes unique to the user. This allows the user to order additional tests if new relevant genetic or other diagnostic tests become available, for example, on a later date, without having to collect sample materials or redraw blood again.

Likened to the second repository or the databank 55, the fourth repository can include a high level of security and/or anonymity. In such cases, for example, only the user or patient has the unique identification code and the corresponding unique password required to access the confidential data and sample materials. No third parties, including healthcare management and insurance companies, employers, and the system or the operator/administer of the system would be able to access these secured repositories. The users thus obtain a high level of anonymity and privacy when taking advantage of the secured electronic (i.e., the second repository) and physical (i.e., the fourth repository) databanks.

The fourth repository or the physical databank may be desirable to some individuals because a diverse array of technologies, such as nucleotide microarrays and protein chips, are becoming available in diagnostic testing, such that multiplexed and parallel testing are made possible. This will in turn facilitate, for example, better prediction of the contribution of multiple genes to the development and progression of certain diseases, since the relative genetic risks may be measured on a broader basis.

### A Health Information System Further Linked Thereto

In another embodiment, the third repository or knowledge base 55 is employed in the methods and systems of this disclosure as discussed above. Advantageously, the knowledge base 55 is relatively comprehensive, and can include, for example, both genetic and non-genetic information of a user, in addition to general biomedical background information on human physiology, diseases, diagnosis and therapies. As such, the third repository may serve as a management system for users' medical records or medical files. In some embodiments, the third repository can mirror the record holders or users as virtual patients and enables the self monitoring and management of their own health conditions.

The third repository thus may be updated regularly to input information on newly available drug treatments, medical expert references, life style recommendations, useful diets, additional relevant testing, and potential participation in clinical trials, among other things. The information update may be performed manually by professional curators or automatically by a computerized process, for example through the network 20.

Further embodiments contemplate the use of professional curators who possess expert medical knowledge; and who scrutinize and filter the information and selectively incorporate into the knowledge base 55. A computerized process is typically implemented as a peripheral application program to the database server 45.It can link to other medical databases and knowledge bases worldwide through the network 20 that are publicly accessible or that are proprietary but for which arrangements have been made to gain access. Further, it can extract and parse new information, compile useful knowledge, and load into the third repository. Such application program may be implemented in any suitable programming language, such as JAVA™, C, C++, Perl, CGI, among others. Also, the knowledge base 55 may employ a relational database management system such as the Oracle database system under the trademark ORACLE or an objected oriented database management system Objectivity/DB under the trademark OBJECTIVITY/DB produced by Objectivity, Inc., among others. The content of the third repository may be cast in a user-friendly language and format, such that it is easily understandable to a layperson with no biomedical background.

Specific embodiments of the disclosure are described by the following examples, which are illustrative but do not limit the disclosure herein in any manner.

### Example 1. Anonymous Administration of Human Diagnostic Tests

The methods and systems of the present disclosure are particular useful for anonymous administration of diagnostic tests that typically require high level of personal security and privacy, e.g., genetic tests, tests for sexually transmitted diseases, paternity tests, and tests for drugs of abuse. According to the embodiments disclosed herein, only the user, or any person or entity he or she entrusts, will have access to the test results. Neither the testing center 110 - which technically performs the test - nor a physician, an Internet provider, a telephone operator, the operator/administer of the system or the system itself would ever know the user's test results. The complete insulation between the first and second repositories and the complete insulation between the first and the fourth repositories, as discussed above, ensure the requisite anonymity and privacy.

Upon registering with the system, the user may order (step 210) the required test through the Internet (e-mail), telephone, fax, letter, postcard, preprinted order card, or in personal. The user may be charged a fee (step 220) before he receives a service package or a kit. The payment may be by a credit card, money order, check, cash, cash on delivery, or through any other electronic paying system.

After receiving the money for the ordered test(s), the system or the operator/administer of the system will forward to the user (step 230), by any delivery or parcel service or registered mail, an individualized service package or kit, which comprises the following items:
(i) a packing list;
(ii) materials for taking the sample, including sample tube, buckle swaps, or a syringe(s), used for collecting sample specimen, and any other tools, fluids, or accessory materials such as antiseptic agents that are necessary for the ordered test;
(iii) instructions for taking the sample, including explanations on how to use the materials and agents provided, how to collect the sample, and how to get professional help for taking the sample;
(iv) information about the ordered test(s) and the range of possible results and their risk implications and possible consequences;
(v) a voucher for the ordered test(s);
(vi) a unique secure and confidential data mailer envelope with four layers of packaging materials, three layers being sealed together as an envelope which contains a printed unique identification code and a barcode adhesive encoded with the same anonymous and unique identification code;
(vii) a sealed data sheet containing the Internet address which linked to the system or the operator/administer of the system - particularly, e.g., the interface of the databank - and a unique identification code assigned, anonymously, to the user, which allows the user to access the test results that will subsequently be stored in the databank 50;
(viii) a self addressed, pre-stamped, prepaid envelope or small parcel to mail the sample and voucher back to the system or the administer/operator of the system without any personal user data; and
(ix) a special adhesive sealing tape to ensure that the envelope cannot be opened without breaking the sealing tape.

The aforementioned unique data mailer envelope may include further specifics as follows: Page 1 contains a unsealed cover sheet which may be taken by a third party mailer or processing entity before shipping to the system or the operator/administer of the system. Page 2 is the face of the sealed unique data mailer envelope; it is of nontransparent packaging material. Page 3 contains a printed unique identification code and a barcode adhesive encoded with the same anonymous and unique identification code; it is of nontransparent packaging material. Page 4 is the backside of the copies of the unique identification code and barcode; it is of nontransparent packaging material. Pages 2, 3, and 4 are sealed together and made easy for the user to check for any broken seal.

After receiving the service package (step 240), the user compares the packing list with the delivery and examines whether the voucher corresponds to his or her order and whether the secure and confidential data mailer envelope is damaged or the sealing is broken. If there is any damage or the sealing is broken, the user may return the package and contact the system or the operator/administer of the system for delivery of a substitute package.

If the package is intact, the user may open the sealing of the data mailer envelop in a secure and private surrounding - typically his or her home (usually his living place). After taking sample specimen, the user may peel off the adhesive barcode label and attach the label on the sample container such as a sample tube containing his or her sample specimen. The user may then put the labeled sample and the voucher into the pre-stamped envelope and seal it with the special adhesive sealing tape and ship it to the designated address (step 250).

Upon receiving the sample, the system or operator/administer of the system may mark a note of arrival in the databank under the user's unique identification code and specify an expected date when the test result may be available. In the case where a sample envelop with a broken seal is received, the operator/administer of the system will mark a note of broken seal instead, under the user's unique identification code. The user can thus be notified and may request a new service package or kit from the system.

The system or the operator/administer of the system may send the bar-coded, anonymous sample to an approved and certified contract laboratory. The test results may be returned from the contract laboratory marked consistently with the user's unique identification code. Thereafter, the test results may be transferred to the databank 50, anonymously (step 260). Only the user is able to access (step 270) the server system, i.e., the interface of the databank, using his unique identification number and a unique password he had created previously that corresponds with the unique identification number.

The test results may be available on the data server, i.e., through the interface of the databank, for a defined time which, for example, corresponds to the user's registration and the fee paid. The user has the option to view orprint his test results (step 280); and, additionally, the user may elect to transfer the results to the system's third repository system, the long-term and comprehensive medical knowledge base 55, as discussed supra.

### Example 2. Genetic Risk Factors In Hemostasis And Deep Vein Thrombosis

Certain individuals are genetically predisposed to develop deep venous thrombosis (DVT) which may lead to fatal lung embolism, especially when subject to immobilization during long-haul air travel. The mortality rate caused by DVT is evidently higher than the mortality rate from aircraft crash. Recent studies indicate that there may be an increased frequency of DVT in the lower limb during long-haul air travel; symptom-less DVT might occur in up to 10 % of long-haul air travelers (The Lancet, 357, 1485-1489 (2001)).

The two most common genetic risk factors in patients with DVT is a single G-to-A base change at nucleotide 1691 (G1691A) in the factor V gene, termed factor V Leiden (FV-Leiden) - and a single G-to-A base change at nucleotide position 20210 (G20210A) within the 3'-untranslated region of the prothrombine (PT) gene. The FV-Leiden mutation appears in 20-60 % of patients with a known DVT history examined for a predisposition to DVT and occurs in approximately 5 % of the western population.

Mutation screening therefore can classify long-haul airline travelers into two categories: those who are required to take precautions to prevent development of DVT (e.g., taking oral anticoagulants or wearing antithrombotic stockings) and those who are not subject to increased risks of DVT. Access to this genetic test is thus of great importance to the potential predisposed individuals. Airlines, for example, also have a strong interest in encouraging their passengers to take such test and become aware of their possible predisposition to DVT.

The test of FVLeiden / PT gene for DVT may be administered in an anonymous and secured way for the travelers according to the present embodiment. A traveler or user may choose to order the test by registering through the Internet, by mail, or by telephone phone, as discussed supra. Billing will take place upon registration. A kit then may be sent to the user which comprises a palatal swap device, a unique identification number, and the information about taking the test and explanation of different results.

The user retrieves the palatal swap, puts the buckle swap device back in the enclosed transport tube and ships the whole package, together with the items described in Example 1, back to the system or the operator/administer of the system. The test results will be available for the user to access via the interface of the databank as described supra, e.g., over the Internet or a telephone. The user may determine his or her risk class based on the test results, according to the classification schema as follows.

FV-Leiden:
Class I: no mutation in both alleles ("homozygous wild type", no increased genetic risk to experience DVT episodes);
Class II: mutation(s) in one of the alleles ("heterozygous wild type / mutant", moderately increased genetic risk to experience DVT episodes, three to eight fold greater risk for thrombosis compared to class I individuals);
Class III: mutation(s) in both alleles ("homozygous mutant", strongly increased genetic risk to experience DVT episode, 100-fold greater risk for thrombosis compared to class I individuals); and

FV-Leiden / Prothrombin:
Class IV: mutations (heterozygous / homozygous) in both genes: very strongly increased genetic risk to experience DVT episodes, 100-fold greater risk for thrombosis compared to class I individuals).

Additional subclasses may be included to account for non-genetic factors, such as environmental and other factors. That is, for example, individuals that belong to classes II, III, and IV may increase their risk levels if they are subject to smoking, oral contraceptives, older age, immobilization, or other coagulation system defects. Also, risk levels may be adjusted for users who had suffered multiple thrombotic events, who have family histories of thrombotic events, who suffered the first thrombotic event at young ages (under 40 to 45), who suffered thrombosis at an unusual anatomic site, e.g. outside the veins of the legs, pelvis, lungs, arms, cerebral veins, or eyes. The users of classes II, III, and IV may be advised to take additional genetic tests on the gene encoding methylen-tetrahydrofolate-reductase (MTHFR), since it is known that a mutation in this gene (MTHFR-A223V mutation) contributes to a multifold additional risk increase to experience thrombotic events.

Other examples on genetic testing include coagulation and thrombotic disorders, hemochromatosis, breast cancer (e.g., BRCA1, BRCA2, others), colon cancer, others cancers, predisposition in drug response genes (e.g., genes for drug metabolizing enzymes like CYP450, and many others), drug receptors (Ah-receptor, vitamin D receptor, LDL receptor), transporters (e.g., serotonin and dopamine transporters) and many others.

Additionally, tests for drugs of abuse, HIV, tests for paternity and other forensic testing may be similarly administered.

It is understood that the above systems are applicable to humans as well as animals, plants, and any other organism for which such testing is desired.

It is further to be understood that the description, specific examples and data, while indicating exemplary embodiments, are given by way of illustration and are not intended to limit the invention(s) described by the appended claims. Various changes and modifications within the invention(s) defined by the appended claims will become apparent to the skilled artisan from the discussion, disclosure and data contained herein, and thus are considered part of the invention(s) described by the appended claims. In the appended claims, the articles such as "a," "an," "the" and the like can mean one or more than one, and are not intended in any way to limit the terms that follow to their singular form, unless expressly noted otherwise. Unless otherwise indicated, any claim which contains the word "or" to indicate alternatives shall be satisfied if one, more than one, or all of the alternatives denoted by the word "or" are present in an embodiment which otherwise meets the limitations of such claim.

## Claims

1. A method for anonymously administering at least one test for one or more users, comprising:
a) registering (210) said one or more users by recording, in a first repository (90), the identity data thereof;
b) forwarding (230) to each of said users, without referencing the identity data thereof, a kit which comprises (i) instructions on and (ii) materials for taking a sample for said test;
c) receiving (240) said sample from each of said users;
d) obtaining, for each of said users, a result of said test on said sample; and
e) recording (270), in a second repository (50), said result for each of said users under a unique identification code,
wherein the first repository (90) and the second repository (50) are insulated from each other.

2. The method of claim 1, wherein said unique identification code is created by each of said users and wherein the receiving further comprises receiving said unique identification code.

3. The method of claim 1 or 2, wherein the test is a diagnostic test for a disorder selected from the group consisting of coagulation and thrombotic disorders, hemochromatosis, cancer, and HIV, or the test is a genetic test for one or more genotypical or phenotypical traits.

4. The method of any one of the above claims, wherein each of said users further creates a unique password which corresponds to the unique identification code, said method further comprising allowing each of said users to access said result under the unique identification code in the second repository (50) using said unique password.

5. A system for anonymously administering at least one test for one or more users, comprising:
a) a first repository (90) having stored therein the identity data of said one or more users, entered by said one of more users upon registration;
b) a kit comprising instructions on and materials for taking a sample for said test, forwarded to each of said users with a unique identification code;
c) a unique password created by each of said users, which corresponds to the unique identification code; and
d) a second repository (55) having stored therein, under the unique identification code for each of said users, a result of said test on said sample, said result being accessible to the user on receipt of the unique password.
wherein the first repository (90) and the second repository (55) are insulated from each other.

6. The system of claim 5, further comprising a first interface (30, 40, 47, 70) capable of receiving the identity data entered by each of said users upon registration, wherein said first interface (30, 40, 47, 70) connects to said first repository (90).

7. The system of claim 6, wherein the first interface (30, 40, 47, 70) comprises a first data transfer system (47) for transferring the identity data to an intermediate storage (70) and a second data transfer system (90) for transferring the identity data from the intermediate storage (70) to a local storage (90).

8. The system of one of claims 5 to 7, further comprising a second interface (40) having a password storage area and capable of providing the result of said test under the unique identification code when the unique password is entered by each of said users, wherein said second interface (40) connects to said second repository (55).

9. The system of one of claims 4 to 8, further comprising a third repository (55) connectable to the second repository for studying the health conditions of the user and/or assessing the health risks of the user.

10. The system of one of claims 5 to 9, further comprising a fourth repository capable of storing, upon an instruction from each of the user, said sample or biological molecules derived therefrom.

11. The system of claim 9, further comprising a classification schema stored in the third repository (55) and based on said test, wherein the classification schema comprises a plurality of risk classes ranked from lowest risk to highest risk, relating to said one or more disorders,

12. The system of claim 11, wherein said kit further comprises information on said classification schema, allowing the user to self-assess the personal health risks relating to said one or more disorders by determining a risk class based on said result of the test.

13. A computer-readable medium (50) having stored therein a data structure, comprising:
one or more first fields containing data representing results of one or more tests performed in accordance with the method of one of claims 1 to 4; and
a second field containing a unique identification code permitting access by the user to said data in said one or more first fields.

14. The computer-readable medium of claim 13, wherein said data structure further comprises a third field containing a unique password created and supplied by the user, said unique password corresponds to said unique identification code thereby permitting access to said data in the one or more first fields.

15. A computer-readable program (15) in a computer system (10) having a user interface, said computer-readable program having
computer instructions for accessing results for one or more tests stored in a databank (50), said one or more tests being performed in accordance with the method of one of claims 1 to 4:
validator (15) for providing to the databank (50) an unique identification code, said unique identification code permitting access by the user to said results in the databank (50); and
display device (10) for displaying on said user interface said results.

16. A test kit for anonymously administering a test in accordance with the method of one of claims 1 to 4, comprising:
instructions for administering said test;
materials for taking said sample for said test;
a unique identification code capable of identifying said sample and permitting access by the user results of said test.
